# NEUE EUROPÄISCHE PATENTSCHRIFT

(11) **EP 2 699 609 B2**
(45) Veröffentlichungstag und Bekanntmachung des Hinweises auf die Entscheidung über den Einspruch: **15.07.2020**
(45) Hinweis auf die Patenterteilung: 18.10.2017
(21) Anmeldenummer: 12711884.2
(22) Anmeldetag: 03.04.2012
(51) Int. Cl.: C08F 220/06, C08F 2/44, A61L 15/00

(54) **VERFAHREN ZUR HERSTELLUNG VON WASSERABSORBIERENDEN POLYMEREN MIT HOHER ABSORPTIONSGESCHWINDIGKEIT**
PROCESS FOR PRODUCING WATER-ABSORBING POLYMERS WITH HIGH ABSORPTION RATE
PROCÉDÉ DE PRODUCTION DE POLYMÈRES ABSORBANT L'EAU AYANT UNE VITESSE D'ABSORPTION ÉLEVÉE

(30) Priorität: 20.04.2011 DE 102011007723
(43) Veröffentlichungstag der Anmeldung: 26.02.2014
(73) Patentinhaber: Evonik Operations GmbH, 45128 Essen (DE)
(72) Erfinder: WATTEBLED, Laurent, 40589 Düsseldorf (DE); LOICK, Christoph, 47918 Tönisvorst (DE); HARREN, Jörg, 52499 Baesweiler (DE); LEININGER, Stefan, 63505 Langenselbold (DE)
(74) Vertreter: Evonik Patent Association
(86) Internationale Anmeldenummer: PCT/EP2012/056019
(87) Internationale Veröffentlichungsnummer: WO 2012/143235

(56) Entgegenhaltungen:
- EP-A1- 0 644 207
- EP-A1- 0 744 435
- US-A- 5 118 719
- US-A- 5 712 316
- US-A1- 2005 137 546
- US-A1- 2005 137 546
- US-A1- 2009 191 408

## Beschreibung

Die vorliegende Erfindung betrifft ein Verfahren zur Herstellung von wasserabsorbierenden Polymeren mit hoher Absorptionsgeschwindigkeit sowie deren Verwendung.

Der aktuelle Trend im Windelaufbau geht dahin, noch dünnere Konstruktionen mit reduziertem Cellulosefaseranteil und erhöhtem Superabsorberanteil herzustellen. Der Vorteil dünnerer Konstruktionen zeigt sich nicht nur in einem verbesserten Tragekomfort, sondern auch in reduzierten Kosten bei Verpackung und Lagerhaltung. Mit dem Trend zu immer dünner werdenden Windelkonstruktionen hat sich das Anforderungsprofil an die Superabsorber deutlich verändert. Von entscheidender Bedeutung ist jetzt die Fähigkeit des Hydrogels zur Flüssigkeitsweiterleitung und -verteilung. Aufgrund der höheren Beladung des Hygieneartikels (Menge an Superabsorber pro Flächeneinheit) darf das Polymer im gequollenen Zustand keine Sperrschicht für nachfolgende Flüssigkeit bilden (Gel-Blocking). Weist das Produkt gute Transporteigenschaften auf, so kann eine optimale Ausnutzung des gesamten Hygieneartikels gewährleistet werden.

Neben der Permeabilität der Superabsorber (angegeben in Form der sogenannten *"Saline Flow Conductivity -* SFC") und dem Absorptionsvermögen unter einer Druckbelastung ist insbesondere auch die Absorptionsgeschwindigkeit der Superabsorberpartikel (angegeben in Menge an absorbierter Flüssigkeit pro Gramm Superabsorber pro Sekunde) ein entscheidendes Kriterium, welches Aussagen darüber ermöglicht, ob ein diesen Superabsorber in großer Konzentration enthaltender absorbierender Kern, welcher nur einen geringen Fluffanteil aufweist, in der Lage ist, bei seinem ersten Kontakt mit Flüssigkeiten diese schnell zu absorbieren (sogenannte *"acquisition").* Diese *"aquisition"* ist bei absorbierenden Kernen mit hohem Superabsorberanteil unter anderem von der Absorptionsgeschwindigkeit des Superabsorbermaterials abhängig.

Aus dem Stand der Technik sind unterschiedliche Schutzrechte bekannt die eine Erhöhung der Absorption ermöglichen sollen. Aus der WO96/17884A1 ist ein wasserabsorbierendes Harz bekannt in dessen Monomerlösung ein festes Treibmittel mit einem Teilchendurchmesser von 1 bis 100 µm eingesetzt wird. Grundsätzlich werden organische Azoverbindungen bevorzugt und hier im Speziellen Acrylsäuresalze von einer Aminogruppe enthaltende Azoverbindungen. Reine Carbonate, Ammoniumnitrid oder Mischungen hieraus können gegebenenfalls verwendet werden.

Nachteilig ist hierbei der schnelle Umsatz der Azoverbindungen als auch das grundsätzliche Dispergieren der kleinen festen Partikel in der Monomerlösung. Größere Partikel können nicht dispergiert werden, ohne dass es zu einer Trennung der unterschiedlichen Partikel in der Dispersion beim Stehenlassen kommt.
Die US 5,118,719 offenbart die Zugabe von Carbonat-Treibmitteln in Form von Carbonaten, Bicarbonaten oder Mischungen dieser, oder das Einführen von gasförmigem Kohlendioxid in die Monomerlösung. Das Treibmittel kann in fester oder gelöster Form in der Dispersion vorliegen und wird kurz vor oder nach der Einleitung der Polymerisation zugefügt. Hierbei werden die Absorptionseigenschaften wie eine höhere Quellrate und Gelfestigkeit erhöht. Nachteilig hierbei ist, dass das Treibmittel bereits vor oder während der Gelbildung aus dem superabsorbierenden Hydrogel entwichen ist, oder größere Blasen bildet die keine mikroporöse Struktur des superabsorbierenden Polymeren gewährleisten.
Die allgemeine Verwendung von Treibmitteln wird ebenfalls in den Druckschriften EP 664207, US5712316, US5399591, US5462972 beschrieben. Hierbei handelt es sich um die Verwendung von meist anorganischen Treibmitteln die Kohlendioxid in Suspensionen freisetzen oder gasförmiges oder festes Kohlendioxid, das der Monomerlösung hinzugefügt werden. Es werden überwiegend Kalium- und Ammoniumverbindungen eingesetzt. Eine Erhöhung der Absorption und der Absorption unter Druck wurde beobachtet.

EP-A-744435, US-A-2005/137546, EP-A-0644207 und US-A-5712316 offenbaren alle die Polymerisation von Acrylat-Superabsorbern mit Carbonat Bläahmitteln in der Monomer Lösung. Allgemein liegt der vorliegenden Erfindung die Aufgabe zu Grunde, die sich aus dem Stand der Technik ergebenden Nachteile zu überwinden.
Es ist insbesondere die Aufgabe der vorliegenden Erfindung, ein Verfahren zur Herstellung eines wasserabsorbierenden Polymeren zur Verfügung zustellen, dass eine verbesserte Quellgeschwindigkeit und schnellere Aufnahme von Flüssigkeiten aufweist unter gleichzeitiger Beibehaltung der Gesamtqualität. Außerdem sollte dieses Verfahren in einfacher Weise durchzuführen sein und ohne den Einsatz von organischen Additiven auskommen.
Es ist insbesondere eine Aufgabe der vorliegenden Erfindung, ein Verfahren bereitzustellen, nach dem wasserabsorbierende Polymere hergestellt werden können, wobei eine besonders hohe Quellgeschwindigkeit gewährleistet werden kann.
Eine weitere Aufgabe der Erfindung besteht vornehmlich darin, ein wasserabsorbierendes Polymer, Verbunde, die derartige wasserabsorbierende Polymere enthalten, und chemische Produkte, die derartige wasserabsorbierende Polymere oder Verbunde enthalten, anzugeben, wobei die wasserabsorbierenden Polymeren eine erhöhte Absorptionsgeschwindigkeit für wässrige Lösungen aufweisen.
Es ist eine weitere Aufgabe der vorliegenden Erfindung ein Verfahren zur Herstellung wasserabsorbierender Polymerer zur Verfügung zu stellen, wobei eine gleichmäßige Verteilung und eine sehr gleichmäßige Wirkung der Blähmittel im Reaktionsraum gewährleistet wird.
Diese Aufgaben werden gelöst durch den Gegenstand der kategoriebildenden Ansprüche. Vorteilhafte Ausgestaltungen und Weiterbildungen, die einzeln oder in Kombination auftreten können, sind Gegenstand der jeweils abhängigen Ansprüche.

Ein Beitrag zur Lösung der eingangs genannten Aufgabe leistet das Verfahren entsprechend des vorliegenden Anspruchs 1 zur Herstellung einer wasserabsorbierenden Polymerzusammensetzung, umfassend die Verfahrenschritte
**(i)** Mischen von
   (α1) 0,1 bis 99,999 Gew.-%, bevorzugt 20 bis 98,99 Gew.-% und besonders bevorzugt 30 bis 98,95 Gew.-% polymerisierbaren, ethylenisch ungesättigten, säuregruppenhaltigen Monomeren oder deren Salze oder polymerisierbaren, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
   (α2) 0 bis 70 Gew.-%, bevorzugt 1 bis 60 Gew.-% und besonders bevorzugt 1 bis 40 Gew.-% polymerisierten, ethylenisch ungesättigten, mit (α1) copolymerisierbaren Monomeren,
   (α3) 0,001 bis 10 Gew.-%, bevorzugt 0,01 bis 7 Gew.-% und besonders bevorzugt 0,05 bis 5 Gew.-% eines oder mehrerer Vernetzer,
   (α4) 0 bis 30 Gew.-%, bevorzugt 1 bis 20 Gew.-% und besonders bevorzugt 5 bis 10 Gew.-% wasserlöslichen Polymeren, sowie
   (α5) 0 bis 20 Gew.-%, bevorzugt 0,01 bis 7 Gew.-% und besonders bevorzugt 0,05 bis 5 Gew.-% eines oder mehrerer Hilfsstoffe, wobei die Summe der Gewichtsmengen (α1) bis (α5) 100 Gew.-% beträgt.
**(ii)** radikalische Polymerisation unter Vernetzung, um ein wasserunlösliches, wässriges unbehandeltes Hydrogel-Polymer zu bilden;
**(iii)** Trocknen des Hydrogel-Polymers
**(iv)** gegebenenfalls Mahlen und Absieben der wasserabsorbierenden Polymers,
**(v)** Oberflächennachvernetzung des gemahlenen Hydrogel-Polymers und
**(vi)** Trocknung und Konfektionierung des wasserabsorbierenden Polymers,
wobei
der wässrigen Monomerlösung, vor der Zugabe des Initiators und dem Start der radikalischen Polymerisation, Blähmittel mit einer Partikelgröße von 100µm bis 900µm zugesetzt werden,
dadurch gekennzeichnet, dass das Verfahren in den Schritten i) und ii) in einem Knetreaktor erfolgt und das Blähmittel aus einem Granulat aus Sodapartikeln besteht.
In einer weiteren Ausführungsform der vorliegenden Erfindung kann im Schritt iii) gegebenenfalls zusätzlich die Zerkleinerung vor dem Trocknen des Hydrogel-Polymers erfolgen.

Bei dem für das erfindungsgemäße Verfahren verwendete Natriumcarbonat handelt es sich um ein Natriumcarbonat, dass eine partikulare Struktur aufweist, die aus mehreren Schichten Natriumcarbonat und Natriumsulfat und/oder einer Hochtemperaturphase aus einem Doppelsalz aus Na₄(SO₄)₁₊ₙ(CO₃)₁₋ₙ mit n von 0 bis 0,5 besteht. Diese Carbonatverbindung hat eine Korngrößenverteilung im Bereich von 100 bis 900 µm. Die Herstellung erfolgt mittels "fluidized bed buildup granulation". Diese Verbindung wird als Reinigungsmittel in Maschinengeschirrreinigern eingesetzt. Im erfindungsgemäßen Verfahren wird bevorzugt reines granuliertes Natriumcarbonat gemäß der WO 2008/012181 eingesetzt.
Bevorzugt wird eine Partikelgröße von 200 µm bis 800 µm und besonders bevorzugt eine Partikelgröße im Bereich von 300 µm bis 700 µm. Die Partikel können eine amorphe oder reguläre dreidimensionale Ausgestaltung aufweisen.
In einer weiteren Ausführungsform kann es sich bei dem verwendeten Blähmittel z.B. um amorphe Partikel handeln, die eine Partikelgröße von 200 µm bis 800 µm, bevorzugt 300 µm bis 700 µm und besonders bevorzugt 400 µm bis 600 µm aufweisen.
Erfindungsgemäß werden die Schritte i) und ii) des erfindungsgemäßen Verfahrens in einem Knetreaktor ausgeführt. Hierbei wird erfindungsgemäß ein Knetreaktor verwendet der mindestens eine gleichlaufende Wellenbewegung ausführt. Bevorzugt werden Knetreaktoren mit mindestens zwei Wellen verwendet.

Vorteilhafterweise wird durch die Verwendung des Granulates als Blähmittel sowie einem Kneter mit mindestens einer Welle eine gleichmäßige Verteilung des Blähmittels in dem entstehenden Hydrogel gewährleistet. Die "schichtenweise" Abtragung wird hervorgerufen durch intensive Knetprozesse und die für diese Zwecke ideale Energiedissipation im Reaktionsinnenraum des Kneters. Hierdurch wird Des Weiteren die gleichmäßige Freisetzung des Kohlendioxids bei der Reaktion ermöglicht. Hierdurch wird vorteilhafterweise ein wasserabsorbierendes Polymer erhalten, das eine höhere Quellgeschwindigkeit aufweist.

Überraschenderweise beobachtet man die positiven Auswirkungen auf die Quellgeschwindigkeit mit dem granulierten Blähmittel nur in Kombination mit dem Kneterpolymerisationsverfahren. Der Einsatz dieser oben beschriebenen granulierten mit einem schichtweisen Aufbau versehenen Carbonatzusammensetzung zur Produktion des Superabsorbers in der Polyband-Technologie führt zu keinem signifikanten Effekt auf die Quellgeschwindigkeit (FSR).

Es ist insbesondere eine Aufgabe der vorliegenden Erfindung, ein Verfahren bereitzustellen, nach dem absorbierende Polymere hergestellt werden können, wobei eine besonders hohe Quellgeschwindigkeit gewährleistet werden kann.

Es ist eine weitere Aufgabe der vorliegenden Erfindung ein Verfahren zur Herstellung wasserabsorbierender Polymer zur Verfügung zustellen die eine gleichmäßige Verteilung der Sodapartikel ermöglichen.

Diese Aufgabe wird durch das erfindungsgemäße Verfahren gelöst bei dem die Verfahrensschritte i) und ii) in einem Knetreaktor mit mindestens einer Welle stattfindet.

Die "schichtenweise" Abtragung wird hervorgerufen durch intensive Knetprozesse und die für diese Zwecke ideale gleichmäßige Energiedissipation im Reaktionsinnenraum.

Überraschenderweise zeigt der Knetreaktor im diskontinuierlichen oder kontinuierlichen Betrieb, mit einem mindestens einem einwelligem System, dass granulierte Natriumcarbonat mit einer Geschwindigkeit von 10 bis 80 Umdrehungen pro min. in der Monomerlösung und dem Verfahrensschritt ii) gleichmäßig eingearbeitet wird. Bevorzugt erfolgt der Verfahrensschritt bevorzugt bei Umdrehungszahlen des Knetreaktors bei 15 bis 60 Umdrehungen pro min., und besonders bevorzugt bei 20 bis 35 Umdrehungen pro min.

Überraschenderweise wurden durch das erfindungsgemäße Verfahren wasserabsorbierende Polymere hergestellt die eine FSR im Bereich von 0,3 bis 0,55 bevorzugt 0,35 bis 0,45 haben.

Überraschenderweise wird bei der Verwendung von granuliertem Soda, festgestellt, dass der erfindungsgemäße Effekt zu einem bestimmten Zeitpunkt, nach der Zugabe von Wasserstoffperoxid und vor Ascorbinsäurezugabe in die Monomerlösung im Knetreaktor dazu führte, dass das resultierende superabsorbierende Polymer höhere Quellgeschwindigkeitswerte aufwies, ohne dass die anderen Kenndaten des wasserabsorbierenden Polymers negativ beeinflusst werden. Dieser Effekt ist mit dem statischen Polybandverfahren in keinem Fall zu beobachten. Die Verwendung von Natriumcarbonat mit unterschiedlicher Dichte (light density und heavy density) führte ebenfalls zu einem erhöhten Wert der Quellgeschwindigkeit, jedoch ist die Steigerung der Quellgeschwindigkeit im Fall des Knetprozesses nicht so ausgeprägt, wie durch die Zugabe des granulierten Natriumcarbonates. Zusätzlich erlaubt die Verwendung des Natriumcarbonates eine Porosität, so dass eine Vergrößerung der Partikeloberfläche ermöglicht wird.

Bevorzugt werden durch die Kombination des Natriumcarbonats und dem einem gleichlaufenden zweiwelligem Kneter in den Schritten i) und ii) des erfindungsgemäßen Verfahrens sowohl der Sauerstoff erniedrigt, die Zerstörung des Granulats stark minimiert, eine Einarbeitung des Carbonates als auch eine gleichmäßige Abgabe an Kohlendioxid gewährleistet.

Die monoethylenisch ungesättigten, säuregruppenhaltigen Monomere (α1) können teilweise oder vollständig, bevorzugt teilweise neutralisiert sein. Vorzugsweise sind die monoethylenisch ungesättigten, säuregruppenhaltigen Monomere zu mindestens 10 Mol-%, besonders bevorzugt zu mindestens 25 bis 50 Mol-% und darüber hinaus bevorzugt zu 50-90 Mol-% neutralisiert. Die Neutralisation der Monomere (α1) kann vor auch nach der Polymerisation erfolgen. Hierbei erfolgt die Teilneutralisierung zu mindestens 10 Mol-%, besonders bevorzugt zu mindestens 25 bis 50 Mol-% und darüber hinaus bevorzugt zu 50-90 Mol-% neutralisiert. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als *"Mixed-Bed Ion-Exchange Absorbent Polymers"* (MBIEA-Polymere) bezeichnet und sind unter anderem in der WO 99/34843 offenbart. Die Offenbarung der WO 99/34843 wird hiermit als Referenz eingeführt und gilt somit als Teil der Offenbarung. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte monoethylenisch ungesättigte, säuregruppenhaltige Monomere (α1) sind Acrylsäure, Methacrylsäure, Ethacrylsäure, α-Chloracrylsäure, α-Cyanoacrylsäure, β-Methylacrylsäure (Crotonsäure), α-Phenylacrylsäure, β-Acryloxypropionsäure, Sorbinsäure, α-Chlorsorbinsäure, 2'-Methylisocrotonsäure, Zimtsäure, p-Chlorzimtsäure, β-Stearylsäure, Itaconsäure, Citraconsäure, Mesaconsäure, Glutaconsäure, Aconitsäure, Maleinsäure, Fumarsäure, Tricarboxyethylen und Maleinsäureanhydrid, wobei Acrylsäure sowie Methacrylsäure besonders und Acrylsäure darüber hinaus bevorzugt sind.

Neben diesen carboxylatgruppenhaltigen Monomeren sind als monoethylenisch ungesättigte, säuregruppenhaltige Monomere (α1) des Weiteren ethylenisch ungesättigte Sulfonsäuremonomere oder ethylenisch ungesättigte Phosphonsäuremonomere bevorzugt.

Als ethylenisch ungesättigte Sulfonsäuremonomere sind Allylsulfonsäure oder aliphatische oder aromatische Vinylsulfonsäuren oder acrylische oder methacrylische Sulfonsäuren bevorzugt. Als aliphatische oder aromatische Vinylsulfonsäuren sind Vinylsulfonsäure, 4-Vinylbenzylsulfonsäure, Vinyltoluolsulfonsäure und Stryrolsulfonsäure bevorzugt. Als Acryl- bzw. Methacrylsulfonsäuren sind Sulfoethyl(meth)acrylat, Sulfopropyl(meth)acrylat, 2-Hydroxy-3-methacryloxypropylsulfonsäure sowie (Meth)Acrylamidoalkylsulfonsäuren wie 2-Acrylamido-2-methylpropansulfonsäure bevorzugt.

Als ethylenisch ungesättigte Phosphonsäuremonomere sind Vinylphosphonsäure, Allylphosphonsäure, Vinylbenzylphosponsäure, (Meth)acrylamidoalkylphosphonsäuren, Acrylamidoalkyldiphosphonsäuren, phosponomethylierte Vinylamine und (Meth)acryl-phosphonsäurederivate bevorzugt.

Als ethylenisch ungesättigte, einen protonierten Stickstoff enthaltende Monomere (α1) sind vorzugsweise Dialkylaminoalkyl(meth)acrylate in protonierter Form, beispielsweise Dimethylaminoethyl(meth)acrylat-Hydrochlorid oder Dimethylaminoethyl(meth)acrylat-Hydro-sulfat, sowie Dialkylaminoalkyl-(meth)acrylamide in protonierter Form, beispielsweise Dimethylaminoethyl(meth)acrylamid-Hydrochlorid, Diemethylaminopropyl(meth)acrylamid-Hydrochlorid, Diemethylaminopropyl(meth)acrylamid-Hydrosulfat oder Dimethylaminoethyl-(meth)acrylamid-Hydrosulfat bevorzugt.

Als ethylenisch ungesättigte, einen quarternierten Stickstoff enthaltende Monomere (α1) sind Dialkylammoniumalkyl(meth)acrylate in quarternisierter Form, beispielsweise Trimethylam-moniumethyl(meth)acrylat-Methosulfat oder Dimethylethylammoniumethyl(meth)acrylat-Etho-sulfat sowie (Meth)acrylamido-alkyldialkylamine in quarternisierter Form, beispielsweise (Meth)acrylamidopro- pyltrimethylammoniumchlorid, Trimethylammoniumethyl(meth)acrylat-Chlorid oder (Meth)acrylamidopropyltrimethylammoniumsulfat bevorzugt.

Als monoethylenisch ungesättigte, mit (α1) copolymerisierbare Monomere (α2) sind Acrylamide und Methacrylamide bevorzugt.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino(meth)- acrylamid, Dimethyl(meth)acrylamid oder Diethyl(meth)acrylamid Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäurester und Methacrylsäurester, wie Methyl(meth)acrylat, Ethyl(meth)acrylat, Propyl(meth)acrylat oder Butyl(meth)acrylat, sowie Vinylacetat, Styrol und Isobutylen bevorzugt.

Erfindungsgemäß bevorzugte Vernetzer (α3) sind Verbindungen, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren (α1) oder (α2) in einer Kondensationsreaktion (=Kondensationsvernetzer), in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV). Dabei wird durch die Verbindungen der Vernetzerklasse I eine Vernetzung der Polymere durch die radikalische Polymerisation der ethylenisch ungesättigten Gruppen des Vernetzermoleküls mit den monoethylenisch ungesättigten Monomeren (α1) oder (α2) erreicht, während bei den Verbindungen der Vernetzerklasse II und den polyvalenten Metallkationen der Vernetzerklasse IV eine Vernetzung der Polymere durch Kondensationsreaktion der funktionellen Gruppen (Vernetzerklasse II) bzw. durch elektrostatische Wechselwirkung des polyvalenten Metallkations (Vernetzerklasse IV) mit den funktionellen Gruppen der Monomere (α1) oder (α2) erreicht wird. Bei den Verbindungen der Vernetzerklasse III erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren (α1) oder (α2).

Bevorzugte Verbindungen der Vernetzerklasse I sind Poly(meth)acrylsäureester, die beispielsweise durch die Umsetzung eines Polyols, wie beispielsweise Ethylenglykol, Propylenglykol, Trimethylolpropan, 1,6-Hexandiol, Glycerin, Pentaerythrit, Polyethylenglykol oder Polypropylenglykol, eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Als Verbindungen der Vernetzerklasse I sind des Weiteren Polyvinylverbindungen, Poly(meth)allyverbindungen, (Meth)acrylsäureester einer Monovinylverbindung oder (Meth)acrylsäureester einer Mono(meth)allyl- verbindung, vorzugsweise der Mono(meth)allylverbindungen eines Polyols oder eines Aminoalkohols, bevorzugt. In diesem Zusammenhang wird auf DE 195 43 366 und DE 195 43 368 verwiesen. Die Offenbarungen werden hiermit als Referenz eingeführt und gelten somit als Teil der Offenbarung.

Als Verbindungen der Vernetzerklasse I seien als Beispiel genannt Alkenyldi(meth)acrylate, beispielsweise Ethylenglykoldi(meth)acrylat, 1,3-Propylenglykoldi(meth)acrylat, 1,4-Butylen-glykoldi(meth)acrylat, 1,3-Butylenglykoldi(meth)acrylat, 1,6-Hexandioldi(meth)acrylat, 1,10-Decandioldi(meth)acrylat, 1,12-Dodecandioldi(meth)acrylat, 1,18-Octadecandioldi(meth)acrylat, Cyclopentandioldi(meth)acrylat, Neopentylglykoldi(meth)acrylat, Methylendi(meth)acrylat oder Pentaerythritdi(meth)acrylat, Alkenyldi(meth)acrylamide, beispielsweise N-Methyldi(meth)acrylamid, N,N'-3-Methylbutylidenbis(meth)acrylamid, N,N'-(1,2-Di-hydroxy-ethylen)bis(meth)acrylamid, N,N'-Hexamethylenbis(meth)acryl-acrylamid oder N,N'-Methylenbis(meth)acrylamid, Polyalkoxydi(meth)acrylate, beispielsweise Diethylenglykoldi-(meth)acrylat, Triethylenglykoldi(meth)acrylat, Tetraethylenglykoldi(meth)acrylat, Dipropylenglykoldi(meth)acrylat, Tripropylenglykoldi(meth)acrylat oder Tetrapropylenglykoldi-(meth)acrylat, Bisphenol-A-di(meth)acrylat, ethoxyliertes Bisphenol-A-di(meth)acrylat, Benzylidindi(meth)acrylat, 1,3-Di(meth)acryloyloxy-propanol-2, Hydrochinondi(meth)acry- lat, Di(meth)acrylatester des vorzugsweise mit 1 bis 30 Mol Alkylenoxid pro Hydroxylgruppe oxyalkylierten, vorzugsweise ethoxylierten Trimethylolpropans, Thioethylenglykoldi(meth)-acrylat, Thiopropylenglykoldi(meth)acrylat, Thiopolyethylenglykoldi(meth)acrylat, Thiopoly-propylenglykoldi(meth)acrylat, Divinylether, beispielsweise 1,4-Butandioldivinylether, Divinylester, beispielsweise Divinyladipat, Alkandiene, beispielsweise Butadien oder 1,6-Hexadien, Divinylbenzol, Di(meth)allylverbindungen, beispielsweise Di(meth)allylphthalat oder Di(meth)allylsuccinat, Homo- und Copolymere von Di(meth)allyldimethylammonium-chlorid und Homo- und Copolymere von Diethyl(meth)allyl- aminomethyl(meth)acrylat-ammoniumchlorid, Vinyl-(meth)acryl-Verbindungen, beispielsweise Vinyl(meth)acrylat, (Meth)allyl-(meth)acryl-Verbindungen, beispielsweise (Meth)allyl(meth)acrylat, mit 1 bis 30 Mol Ethylenoxid pro Hydroxylgruppe ethoxyliertes (Meth)allyl(meth)acrylat, Di(meth)allylester von Polycarbonsäuren, beispielsweise Di(meth)allylmaleat, Di(meth)allyfumarat, Di(meth)allylsuccinat oder Di(meth)allylterephthalat, Verbindungen mit 3 oder mehr ethylenisch ungesättigten, radikalisch polymerisierbaren Gruppen wie beispielsweise Glycerintri(meth)acrylat, (Meth)acrylatester des mit vorzugsweise 1 bis 30 Mol Ethylenoxid pro Hydroxylgruppe oxyethylierten Glycerins, Trimethylolpropantri(meth)acrylat, Tri(meth)acrylatester des vorzugsweise mit 1 bis 30 Mol Alkylenoxid pro Hydroxylgruppe oxyalkylierten, vorzugsweise ethoxylierten Trimethylolpropans, Trimethacrylamid, (Meth)allylidendi(meth)acrylat, 3-Allyloxy-1,2-propandioldi(meth)acrylat, Tri- (meth)allylcyan-urat, Tri(meth)allylisocyanurat, Pentaerythrittetra(meth)acrylat, Pentaerythrittri(meth)acrylat, (Meth)acrylsäureester des mit vorzugsweise 1 bis 30 Mol Ethylenoxid pro Hydroxylgruppe oxyethylierten Pentaerythrits, Tris(2-hydroxyethyl)isocyanurattri(meth)acrylat, Trivinyltrimellitat, Tri(meth)allylamin, Di(meth)allylalkylamine, beispielsweise Di(meth)allylmethylamin, Tri-(meth)allylphosphat, Tetra(meth)allylethylendiamin, Poly(meth)allylester, Tetra(meth)allyloxi-ethan oder Tetra(meth)allylammoniumhalide.

Als Verbindung der Vernetzerklasse II sind Verbindungen bevorzugt, die mindestens zwei funktionelle Gruppen aufweisen, die in einer Kondensationsreaktion (=Kondensaionsvernetzer), in einer Additionsreaktion oder in einer Ringöffnungsreaktion mit den funktionellen Gruppen der Monomere (α1) oder (α2), bevorzugt mit Säuregruppen, der Monomeren (α1), reagieren können. Bei diesen funktionellen Gruppen der Verbindungen der Vernetzerklasse II handelt es sich vorzugsweise um Alkohol-, Amin-, Aldehyd-, Glycidyl-, Isocyanat-, Carbonat- oder Epichlorfunktionen.

Als Verbindung der Vernetzerklasse II seien als Beispiele genannt Polyole, beispielsweise Ethylenglykol, Polethylenglykole wie Diethylenglykol, Triethylenglykol und Tetraethylenglykol, Propylenglykol, Polypropylenglykole wie Dipropylenglykol, Tripropylenglykol oder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin, Diethanolamin, Triethanolamin oder Propanolamin, Polyaminverbindungen, beispielsweise Ethylendiamin, Diethylentriaamin, Triethylentetraamin, Tetraethylenpentaamin oder Pentaethylenhexaamin, Polyglycidylether-Verbindungen wie Ethylenglykoliglycidylether, Polyethylenglykoldiglycidylether, Glycerindiglycidylether, Glycerinpolyglycidyether, Pentareritritpolyglycidylether, Propylenglykoldiglycidylether Polypropylenglykoldiglycidylether, Neopentylglykoldiglycidylether, Hexandiolglycidylether, Trimethylolpropanpolyglycidylether, Sorbitolpolyglycidylether, Phtahlsäurediglycidylester, Adipinsäurediglycidylether, 1,4-Phenylen-bis(2-oxazolin), Glycidol, Polyisocyanate, vorzugsweise Diisocyanate wie 2,4-Toluoldiisocyanat und Hexamethylendiisocyanat, Polyaziridin-Verbindungen wie 2,2-Bishydroxymethylbutanol-tris[3-(1 -aziridinyl)propionat], 1,6-Hexamethylendiethylenharnstoff und Diphenylmethan-bis-4,4'-N,N'-diethylenharnstoff, Halogenperoxide beispielsweise Epichlor- und Epibromhydrin und α-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, polyquartäre Amine wie Kondensationsprodukte von Dimethylaminen und Epichlorhydrin. Als Verbindungen der Vernetzerklasse II sind des Weiteren Polyoxazoline wie 1,2-Ethylenbisoxazolin, Vernetzer mit Silangruppen wie γ-Glycidoxypropyltrimethoxysilan und γ-Aminopropyltrimethoxysilan, Oxazolidinone wie 2-Oxazolidinon, Bis- und Poly-2-oxazolidinone und Diglykolsilikate bevorzugt.

Als Verbindungen der Klasse III sind hydroxyl- oder aminogruppenhaltige Ester der (Meth)acrylsäure, wie beispielsweise 2-Hydroxyethyl(meth)acrylat und 2-Hydroxypropyl-(meth)acrylat sowie hydroxyl- oder aminogruppenhaltige (Meth)acrylamide oder Mono(meth)allylverbindungen von Diolen bevorzugt.

Die polyvalenten Metallkationen der Vernetzerklasse IV leiten sich vorzugsweise von ein- oder mehrwertigen Kationen ab, die einwertigen insbesondere von Alkalimetallen, wie Kalium, Natrium, Lithium, wobei Lithium bevorzugt wird. Bevorzugte zweiwertige Kationen leiten sich von Zink, Beryllium, Erdalkalimetallen, wie Magnesium, Calcium, Strontium ab, wobei Magnesium bevorzugt wird. Weiter erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z. B. AlCl₃ × 6H₂O, NaAl(SO₄)₂ × 12 H₂O, KAl(SO₄)₂ × 12 H₂O oder Al₂(SO₄)₃×14-18 H₂O eingesetzt. Besonders bevorzugt werden Al₂(SO₄)₃ und seine Hydrate als Vernetzer der Vernetzungsklasse IV verwendet.

Die im erfindungsgemäßen Verfahren eingesetzten Superabsorberteilchen sind vorzugsweise durch Vernetzer der folgenden Vernetzerklassen bzw. durch Vernetzer der folgenden Kombinationen von Vernetzerklassen vernetzt: I, II, III, IV, I II, I III, I IV, I II III, I II IV, I III IV, II III IV, II IV oder III IV. Die vorstehenden Kombinationen von Vernetzerklassen stellen jeweils eine bevorzugte Ausführungsformen von Vernetzern eines im erfindungsgemäßen Verfahren eingesetzten Superabsorberteilchens dar.

Weitere bevorzugte Ausführungsformen der im erfindungsgemäßen Verfahren eingesetzten Superabsorberteilchen sind Polymere, die durch einen beliebigen der vorstehend genannten Vernetzer der Vernetzerklassen I vernetzt sind. Unter diesen sind wasserlösliche Vernetzer bevorzugt. In diesem Zusammenhang sind N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (α4) können in den Superabsorberteilchen wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol.

Die wasserlöslichen Polymere, vorzugsweise synthetische wie Polyvinylalkohol, können auch als Pfropfgrundlage für die zu polymerisierenden Monomeren dienen.

Als Hilfsstoffe (α5) sind in den Polymeren, organische oder anorganische Partikel wie beispielsweise Geruchsbinder, insbesondere Zeolithe oder Cyclodextrine, Hautpflegesubstanzen, oberflächenaktive Mittel oder Antioxidantien enthalten.

Zu den bevorzugten organischen Hilfsstoffen gehören Cyclodextrine oder deren Derivate sowie Polysaccharide. Außerdem sind Cellulose und Cellulosederivate wie CMC, Celluloseether bevorzugt. Als Cyclodextrine oder Cyclodextrinderivate sind dabei diejenigen Verbindungen bevorzugt, die in DE-A-198 25 486 auf der Seite 3, Zeile 51 bis Seite 4, Zeile 61 offenbart sind. Der vorstehend genannte Abschnitt dieser veröffentlichten Patentanmeldung wird hiermit als Referenz eingeführt und gilt als Teil der Offenbarung der vorliegenden Erfindung. Besonders bevorzugte Cyclodextrine sind nicht derivatisierte α-, ß-, γ- oder δ-Cyclodextrine.

Als anorganische partikuläre Hilfsstoffe können alle Materialen eingesetzt werden, welches üblicherweise zur Modifizierung der Eigenschaften wasserabsorbierender Polymere eingesetzt wird. Zu den bevorzugten anorganischen Hilfsstoffen gehören Sulfate wie Na₂SO₄, Lactate wie etwa Natriumlactat, Silikate, insbesondere Gerüstsilikate wie Zeolithe oder Silikate, die durch Trocknung wässriger Kieselsäurelösungen oder Kieselsolen erhalten wurden, beispielsweise die kommerziell erhältlichen Produkte wie Fällungskieselsäuren und pyrogene Kieselsäuren, beispielsweise Aerosile mit einer Teilchengröße im Bereich von 5 bis 50 nm, vorzugsweise im Bereich von 8 bis 20 nm wie "Aerosil 200" der Evonik Industries AG, Aluminate, Titandioxide, Zinkoxide, Tonmaterialien und weitere dem Fachmann geläufige Mineralien sowie kohlenstoffhaltige anorganische Materialien.

Bevorzugte Silikate sind alle natürlichen oder synthetischen Silikate, die in Hollemann und Wiberg, Lehrbuch der Anorganischen Chemie, Walter de Gruyter-Verlag, 91.-100. Auflage, 1985 auf den Seiten 750 bis 783, als Silikate offenbart sind. Der vorstehend genannte Abschnitt dieses Lehrbuchs wird hiermit als Referenz eingeführt und gilt als Teil der Offenbarung der vorliegenden Erfindung.

Besonders bevorzugt Silikate sind die Zeolithe. Als Zeolithe können alle dem Fachmann bekannten synthetischen oder natürlichen Zeolithe eingesetzt werden. Bevorzugte natürliche Zeolithe sind Zeolithe aus der Natrolith-Gruppe, der Harmoton-Gruppe, der Mordenit-Gruppe, der Chabasit-Gruppe, der Faujasit-Gruppe (Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabasit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmoton, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS.

Als Zeolithe können Zeolithe des so genannten "mittleren" Typs eingesetzt werden, bei denen das SiO₂/AlO₂-Verhältnis kleiner als 10 ist, besonders bevorzugt liegt das SiO₂/AlO₂-Verhältnis dieser Zeolithe in einem Bereich von 2 bis 10. Neben diesen "mittleren" Zeolithen können weiterhin Zeolithe des "hohen" Typs eingesetzt werden, zu denen beispielsweise die bekannten "Molekularsieb"-Zeolithe des Typs ZSM sowie ß-Zeolith gehören. Diese "hohen" Zeolithe sind vorzugsweise durch ein SiO₂/AlO₂-Verhältnis von mindestens 35, besonders bevorzugt von einem SiO₂/AlO₂-Verhältnis in einem Bereich von 200 bis 500 gekennzeichnet.

Als Aluminate werden vorzugsweise die in der Natur vorkommenden Spinelle, insbesondere gewöhnlicher Spinell, Zinkspinell, Eisenspinell oder Chromspinell eingesetzt.

Bevorzugte Titandioxide ist das reine Titandioxid in den Kristallformen Rutil, Anatas und Brookit, sowie eisenhaltige Titandioxide wie beispielsweise Ilmenit, calciumhaltige Titandioxide wie Titanit oder Perowskit.

Bevorzugte Tonmaterialien sind diejenigen, die in Hollemann und Wiberg, Lehrbuch der Anorganischen Chemie, Walter de Gruyter-Verlag, 91.-100. Auflage, 1985 auf den Seiten 783 bis 785, als Tonmaterialien offenbart sind. Besonders Der vorstehend genannte Abschnitt dieses Lehrbuchs wird hiermit als Referenz eingeführt und gilt als Teil der Offenbarung der vorliegenden Erfindung. Besonders bevorzugte Tonmaterialien sind Kaolinit, Illit, Halloysit, Montmorillonit sowie Talk.

Weiterhin sind als anorganische Feinteilchen die Metallsalze der Mono-, Oligo- und Polyphosphorsäuren erfindungsgemäß bevorzugt. Hierunter sind insbesondere die Hydrate bevorzugt, wobei die Mono- bis Deca-Hydrate und Tri-Hydrate besonders bevorzugt sind. Als Metalle kommen insbesondere Alkali- und Erdalkalimetalle in Betracht, wobei die Erdalkalimetalle bevorzugt sind. Hierunter sind Mg und Ca bevorzugt und Mg besonders bevorzugt. Im Zusammenhang mit Phosphaten, Phosphorsäuren und deren Metallverbindungen wird auf Hollemann und Wiberg, Lehrbuch der Anorganischen Chemie, Walter de Gruyter-Verlag, 91.-100. Auflage, 1985, auf den Seiten 651 bis 669 verwiesen. Der vorstehend genannte Abschnitt dieses Lehrbuchs wird hiermit als Referenz eingeführt und gilt als Teil der Offenbarung der vorliegenden Erfindung.

Bevorzugte kohlenstoffhaltige, jedoch nicht organische Hilfsstoffe sind diejenigen reinen Kohlenstoffe, die in Hollemann und Wiberg, Lehrbuch der Anorganischen Chemie, Walter de Gruyter-Verlag, 91.-100. Auflage, 1985 auf den Seiten 705 bis 708 als Graphite genannt sind. Der vorstehend genannte Abschnitt dieses Lehrbuchs wird hiermit als Referenz eingeführt und gilt als Teil der Offenbarung der vorliegenden Erfindung. Besonders bevorzugte Graphite sind künstliche Graphite wie beispielsweise Koks, Pyrographit, Aktivkohle oder Ruß.

Die im erfindungsgemäßen Verfahren eingesetzten Superabsorberteilchen sind vorzugsweise dadurch erhältlich, dass zunächst aus den vorgenannten Monomeren und Vernetzern ein wasserabsorbierendes Polymer (P) in partikulärer Form hergestellt wird. Die Herstellung dieses als Ausgangsmaterial für die Superabsorberteilchen dienenden Polymers (P) erfolgt beispielsweise durch Massepolymerisation, die vorzugsweise in Knetreaktoren wie Extrudern erfolgt, Lösungspolymerisation, Spraypolymerisation, inverse Emulsionspolymerisation oder inverse Suspensionspolymerisation. Bevorzugt wird die Lösungspolymerisation in Wasser als Lösemittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818. Die Offenbarungen werden hiermit als Referenz eingeführt und gelten somit als Teil der Offenbarung.

Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Hierzu gehören thermische Katalysatoren, Redoxkatalysatoren und Photoinitiatoren, deren Aktivierung durch energiereiche Strahlung erfolgt. Die Polymerisationsinitiatoren können dabei in einer Lösung erfindungsgemäßer Monomere gelöst oder dispergiert enthalten sein. Bevorzugt ist der Einsatz wasserlöslicher Katalysatoren.

Als thermische Initiatoren kommen sämtliche dem Fachmann bekannte, unter Temperatureinwirkung in Radikale zerfallende Verbindungen in Betracht. Besonders bevorzugt sind dabei thermische Polymerisationsinitiatoren mit einer Halbwertszeit von weniger als 10 Sekunden, darüber hinaus bevorzugt von weniger als 5 Sekunden bei weniger als 180 °C, darüber hinaus bevorzugt bei weniger als 140°C. Dabei sind Peroxide, Hydroperoxide, Wasserstoffperoxid, Persulfate sowie Azoverbindungen besonders bevorzugte thermische Polymerisationsinitiatoren. In manchen Fällen ist es vorteilhaft, Mischungen verschiedener thermischer Polymerisationsinitiatoren zu verwenden. Unter diesen Mischungen sind die aus Wasserstoffperoxid und Natrium- oder Kaliumperoxodisulfat bevorzugt, die in jedem denkbaren Mengenverhältnis eingesetzt werden können. Geeignete organische Peroxide sind vorzugsweise Acetylacetonperoxid, Methylethylketonperoxid, Benzoylperoxid, Lauroylperoxid, Acetylperoxid, Capyrlperoxid, Isopropylperoxydicarbonat, 2-Ethylhexylperoxydicarbonat, t-Butylhydroperoxid, Cumolhydroperoxid, t-Amylperpivalat, t-Butylperpivalat, t-Butylperneohexonat, t-Butylisobutyrat, t-Butylper-2-ethylhexenoat, t-Butylperisononanoat, t-Butylpermaleat, t-Butylperbenzoat, t-Butyl-3,5,5-tri-methylhexanoat und Amylperneodekanoat. Weiterhin sind als thermische Polymerisationsinitiatoren bevorzugt: Azo-Verbindungen, wie Azobisisobutyronitrol, Azobisdimethylvaleronitril, 2,2'-Azobis-(2-amidinopropan)dihydrochlorid, Azo-bis-amidinopropan-dihydrochlord, 2,2'-Azobis-(N,N-dimethylen)isobutyramidin-dihydrochlorid, 2- (Carbamoylazo)- isobutyronitril und 4,4'-Azobis-(4-cyanovaleriansäure). Die genannten Verbindungen werden in üblichen Mengen eingesetzt, vorzugsweise in einem Bereich von 0,01 bis 5, bevorzugt von 0,1 bis 2 Mol-%, jeweils bezogen auf die Menge der zu polymerisierenden Monomere.

Die Redoxkatalysatoren enthalten als oxidische Komponente mindestens eine der oben angegebenen Perverbindungen und als reduzierende Komponente vorzugsweise Ascorbinsäure, Glukose, Sorbose, Manose, Ammonium- oder Alkalimetall-hydrogensulfit, - sulfat, -thiosulfat, -hyposulfit oder -sulfid, Metallsalze, wie Eisen-II-ionen oder Silberionen oder Natriumhydroxymethylsulfoxylat. Vorzugsweise wird als reduzierende Komponente des Redoxkatalysators Ascorbinsäure oder Natriumpyrosulfit verwendet. Bezogen auf die bei der Polymerisation eingesetzte Menge an Monomeren wird 1×10⁻⁵ bis 1 Mol-% der reduzierenden Komponente des Redoxkatalysators und 1×10⁻⁵ bis 5 Mol-% der oxidierenden Komponente des Redoxkatalysators eingesetzt. Anstelle der oxidierenden Komponente des Redoxkatalysators, oder in Ergänzung zu diesem, können ein oder mehrere, vorzugsweise wasserlösliche, Azoverbindungen verwendet werden.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte α-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)malein- imid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclo-hexanon und 2,6-Bis-(p-azidobenzyliden)-4-methylcyclohexanon. Die Photoinitiatoren werden, falls sie eingesetzt werden, üblicherweise in Mengen von 0,01 bis 5 Gew.-%, bezogen auf die zu polymerisierenden Monomeren angewendet.

Bevorzugt wird erfindungsgemäß ein Redoxsystem bestehend aus Wassersoffperoxid, Natriumperoxodisulfat und Ascorbinsäure eingesetzt. In der Regel wird die Polymerisation mit den Initiatoren in einem Temperaturbereich von 0°C bis 90°C initiiert.

Die Polymerisationsreaktion kann durch einen Initiator oder durch mehrere, zusammenwirkende Initiatoren ausgelöst werden. Weiterhin kann die Polymerisation derart durchgeführt werden, dass man zunächst ein oder mehrere Redoxinitiatoren zusetzt. Im weiteren Polymerisationsverlauf werden dann zusätzlich thermische Initiatoren oder Photoinitiatoren appliziert, wobei im Falle von Photoninitiatoren die Polymerisationsreaktion dann durch die Einwirkung energiereicher Strahlung initiiert wird. Auch die umgekehrte Reihenfolge, also die anfängliche Initiierung der Reaktion mittels energiereicher Strahlung und Photoinitiatoren oder thermischen Initiatoren und eine im weiteren Polymerisationsverlauf erfolgende Initiierung der Polymerisation mittels eines oder mehrere Redoxinitiatoren ist denkbar.

Um die so erhaltenen Polymere (P) in eine partikuläre Form zu überführen, können diese nach ihrer Abtrennung aus der Reaktionsmischung zunächst bei einer Temperatur in einem Bereich von 20 bis 300°C, bevorzugt in einem Bereich von 50 bis 250°C und besonders bevorzugt in einem Bereich von 100 bis 200°C bis hin zu einem Wassergehalt von weniger als 40 Gew.-%, bevorzugt von weniger als 20 Gew.-% und darüber hinaus bevorzugt von weniger als 10 Gew.-%, jeweils bezogen auf das Gesamtgewicht des Polymers (P), getrocknet werden. Die Trocknung erfolgt vorzugsweise in dem Fachmann bekannten Öfen oder Trockner, beispielsweise in Bandtrocknern, Hordentrocknern, Drehrohröfen, Wirbelbetttrocknern, Tellertrocknern, Paddeltrocknern oder Infrarottrocknern.

Gemäß der vorliegenden Erfindung erfolgt das Zerkleinern dabei vorzugsweise durch Trockenmahlen, vorzugsweise durch Trockenmahlen in einer Hammermühle, einer Stiftmühle, einer Kugelmühle oder einer Walzenmühle.

In einer bevorzugten Ausführungsform der erfindungsgemäßen Verfahren werden als Superabsorberteilchen Partikel eingesetzt, die einen Innenbereich und einen den Innenbereich begrenzenden Oberflächenbereich aufweisen und wobei der Oberflächenbereich eine andere chemische Zusammensetzung als der Innenbereich aufweist oder sich in einer physikalischen Eigenschaft vom Innenbereich unterscheidet. Physikalische Eigenschaften, in denen sich der Innenbereich vom Oberflächenbereich unterscheidet, sind beispielsweise die Ladungsdichte oder der Vernetzungsgrad.

Diese einen Innenbereich und einen den Innenbereich begrenzenden Oberflächenbereich aufweisenden Superabsorberteilchen sind vorzugsweise dadurch erhältlich, dass oberflächennahe, reaktive Gruppen der Superabsorberteilchen vor oder nach ihrer Abtrennung von den übrigen Partikeln des partikulären Polymers (P) nachvernetzt werden. Diese Nachvernetzung kann thermisch, photochemisch oder chemisch erfolgen.

Als Nachvernetzer bevorzugt sind die im Zusammenhang mit den Vernetzern (α3) genannten Verbindungen der Vernetzerklasse II und IV.

Unter diesen Verbindungen sind als Nachvernetzer besonders bevorzugt Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on. Besonders bevorzugt wird Ethylencarbonat als Nachvernetzer eingesetzt.

Bevorzugte Ausführungsformen der Superabsorberteilchen sind diejenigen, die durch Vernetzer der folgenden Vernetzerklassen bzw. durch Vernetzer der folgenden Kombinationen von Vernetzerklassen nachvernetzt sind: II, IV und II IV.

Vorzugsweise wird der Nachvernetzer in einer Menge in einem Bereich von 0,01 bis 30 Gew.-%, besonders bevorzugt in einer Menge in einem Bereich von 0,1 bis 20 Gew.-% und darüber hinaus bevorzugt in einer Menge in einem Bereich von 0,3 bis 5 Gew.-%, jeweils bezogen auf das Gewicht der superabsorbierenden Polymere bei der Nachvernetzung eingesetzt.

Es ist ebenfalls bevorzugt, dass die Nachvernetzung dadurch erfolgt, dass ein Fluid F umfassend ein Lösemittel, vorzugsweise Wasser, mit Wasser mischbare organische Lösemittel wie etwa Methanol oder Ethanol oder Mischungen aus mindestens zwei davon, sowie den Nachvernetzer mit dem Aussenbereich der Polymerteilchen bei einer Temperatur in einem Bereich von 30 bis 300°C, besonders bevorzugt in einem Bereich von 100 bis 200°C in Kontakt gebracht werden. Das in Kontakt bringen erfolgt dabei vorzugsweise durch Aufsprühen des Fluids F auf die Polymerteilchen und anschließendes Mischen der mit dem Fluid F in Kontakt gebrachten Polymerteilchen. Dabei ist der Nachvernetzer in dem Fluid F vorzugsweise in einer Menge in einem Bereich von 0,01 bis 20 Gew.-%, besonders bevorzugt in einer Menge in einem Bereich von 0,1 bis 10 Gew.-%, bezogen auf das Gesamtgewicht des Fluids F, enthalten. Es ist weiterhin bevorzugt, dass das Fluid F in einer Menge in einem Bereich von 0,01 bis 50 Gew.%, besonders bevorzugt in einer Menge in einem Bereich von 0,1 bis 30 Gew.-%, jeweils bezogen auf das Gewicht der Polymerteilchen, mit den Polymerteilchen in Kontakt gebracht wird.

Das in den erfindungsgemäßen Verfahren im Verfahrensschritt (vi) eingesetzte Fluid umfasst vorzugsweise ein Lösemittel sowie das vernetzbare, nicht vernetzte Polymer. Als Lösemittel werden vorzugsweise Wasser oder polare, mit Wasser mischbare Lösemittel wie Aceton, Methanol, Ethanol, 2-Propanol oder Mischungen aus mindestens zwei davon eingesetzt. Dabei kann das nicht vernetzte Polymer in dem Lösemittel gelöst oder dispergiert sein.

Als Kondensationsreaktionen kommen vorzugsweise die Bildung von Ester-, Amid-, Imid- oder Urethanbindungen in Betracht, wobei die Bildung von Esterbindung bevorzugt ist. Diese Esterbindungen werden vorzugsweise durch die Reaktion einer OH-Gruppe des vernetzbaren, nicht vernetzten Polymers mit einer Säuregruppe des Superabsorberteilchens oder mit einer Säuregruppe des vernetzbaren, nichtvernetzten Polymers gebildet.

Die säuregruppenhaltigen Monomere (β1) sind vorzugsweise zu mindestens 10 Mol-%, besonders bevorzugt zu mindestens 20 Mol-%, darüber hinaus bevorzugt zu mindestens 40 Mol-% und weiterhin bevorzugt im Bereich von 45 bis 80 Mol-% neutralisiert. Die Neutralisation der Monomere kann vor, während oder erst nach der Herstellung des vernetzbaren, nichtvernetzten Polymers erfolgen. Die Neutralisierung erfolgt dabei vorzugsweise mit denjenigen Basen, die bereits im Zusammenhang mit der Neutralisation der säuregruppentragenden Monomere (α1) genannt wurden. Neben den dort genannten Basen werden zur Neutralisation der nicht vernetzten Polymere vorzugsweise auch Basen eingesetzt, die Ammonium, Calcium oder Magnesium als Kationen enthalten. In diesem Zusammenhang bevorzugte Basen sind Ammoniumcarbonat, Ammoniak, Calciumcarbonat, Calciumhydroxid, Magnesiumhydroxid und Magnesiumcarbonat.

Als Monomere (β1) und (β2) werden vorzugsweise diejenigen Monomere eingesetzt, die auch als bevorzugte Monomere (α1) bzw. (α2) eingesetzt werden.

Grundsätzlich kommen als Monomere (M) bzw (β3) alle dem Fachmann geeigneten Monomere, insbesondere die der Vernetzerklasse III, in betracht. Bevorzugte Monomere (β3) sind die Umsetzungsprodukte von gesättigten aliphatischen, cycloalphatischen, aromatischen Alkoholen, Aminen oder Thiolen mit ethylenisch ungesättigten Carbonsäuren, reaktiven Carbonsäurederivaten oder Allylhalogeniden. Als Beispiele seien in diesem Zusammenhang genannt: (Meth)allylalkohol, (Meth)allylamin, hydroxyl- oder aminogruppenhaltige Ester der (Meth)acrylsäure, wie Hydroxyalkylacrylate, insbesondere Hydroxymethyl(meth)acrylat, 2-Hydroxyethyl(meth)acrylat oder 2-Hydroxypropyl (meth)acrylat, Aminomalkyl(meth)acrylate, insbesondere Aminomethyl(meth)acrylat, 2-Aminoethyl(meth)acrylat oder 2-Aminopropyl(meth)acrylat, Mono(meth)allylverbindungen von Polyolen, vorzugsweise von Diolen wie beispielsweise Polyethylenglykole oder Polypropylenglykole, sowie Glycidylalkyl(meth)acrylate wie Glycidyl-(meth)acrylat.

Besonders bevorzugte vernetzbare, nicht vernetzte Polymere, die in den erfindungsgemäßen Verfahren eingesetzt werden, sind diejenigen Polymere, die auf 1 bis 80 Gew.-%, besonders bevorzugt auf 1 bis 60 Gew.-% und darüber hinaus bevorzugt auf 1 bis 20 Gew.-% (Meth)acrylamid und 20 bis 99 Gew.-%, besonders bevorzugt auf 40 bis 99 Gew.-% und darüber hinaus bevorzugt auf 80 bis 99 Gew.-%, jeweils bezogen auf das Gesamtgewicht des nicht vernetzten Polymers, auf (Meth)acrylsäure basieren, wobei die (Meth)acrylsäure vorzugsweise teilneutralisiert ist.

Es ist weiterhin bevorzugt, dass das in dem erfindungsgemäßen Verfahren eingesetzte Fluid neben dem Lösemittel und dem vernetzbaren, nicht vernetzten Polymer einen weiteren, externen Vernetzer umfasst. Dies gilt insbesondere dann, wenn die vernetzbaren, nicht vernetzten Polymere keine Monomere (M) bzw. (β3) beinhalten. Als weiterer externer Vernetzer sind dabei diejenigen der Vernetzerklassen II und IV bevorzugt, die bereits im Zusammenhang mit den Vernetzern (α3) genannt wurden. Besonders bevorzugte weitere Vernetzer sind diejenigen, die als besonders bevorzugte Vernetzer der Klassen II und IV im Zusammenhang mit den Monomeren (α3) genannt wurden. Es ist in diesem Zusammenhang weiterhin bevorzugt, dass das Fluid den weiteren externen Vernetzer in einer Menge in einem Bereich von 0,01 bis 30 Gew.-%, bevorzugt in einem Bereich von 0,1 bis 15 Gew.-% und besonders bevorzugt in einem Bereich von 0,2 bis 7 Gew.-%, bezogen auf das Gewicht des nicht vernetzten Polymers, enthält.

Bevorzugte Additive sind Substanzen, welche die Brüchigkeit der durch das erfindungsgemäße Verfahren hergestellten Superabsorberpartikel reduzieren, wie etwa Polyethylenglykol, Polypropylenglykol, gemischte Polyalkoxylate, auf Polyole wie Glycerin, Trimethylolpropan oder Butandiol basierende Polyalkoxylate, Tenside mit einem HLB von mehr als 10 wie Alkylpolyglucoside oder ethoxylierte Zuckerester, beispielsweise Polysorbate unter dem Handelsnamen Tween von ICI. Diese Additive wirken teilweise zugleich auch als weitere Vernetzer, wie zum Beispiel Polyethylenglykol, Polypropylenglykol, Trimethylolpropan oder Butandiol.

Weiterhin als Additive bevorzugt sind Mittel, welche die Härte der durch das erfindungsgemäße Verfahren hergestellten Superabsorberpartikel reduzieren, wie etwa kationische Tenside wie Alkyltrimethylammoniumchlorid, Dialkyldimethylammoniumchlorid, Dimethylstearylammoniumchlorid, Alkylbenzyldimethylammoniumchlorid oder die entsprechenden Methylsulfate, quaternäre Tallölfettsäure-Imidazoliniummethosulfate. Diese Additive werden vorzugsweise in Mengen in einem Bereich von 0 bis 5 Gew. %, besonders bevorzugt in einem Bereich von 0,5 bis 4 Gew.-%, bezogen auf das Gewicht des nicht vernetzten Polymers, eingesetzt. Die Additive können dabei entweder vor oder nach der Polymerisation zugesetzt werden. Sie binden die Polycarboxylate durch Anionen-Kationen-Wechselwirkung und bewirken somit den Erweichungseffekt. Sie bewirken gleichzeitig eine Verbesserung der Absorptionsfähigkeit für wässrige Flüssigkeiten. Ein anderer Vorteil der Substanzen ist ihre biozide Wirkung, die einen ungewollten Abbau der Quellungsmittel verhindert. Diese Eigenschaft ist für manche Anwendungen besonders wichtig.

Als Additive sind des weiteren Trennmittel bevorzugt, wie etwa anorganische oder organische pulverförmige Trennmittel. Diese Trennmittel werden vorzugsweise in Mengen in einem Bereich von 0 bis 2 Gew.-%, besonders bevorzugt in einem Bereich von 0,1 bis 1,5 Gew.-%, bezogen auf das Gewicht des vernetzten Polymers, eingesetzt. Bevorzugte Trennmittel sind Holzmehl, Pulp Fasern, pulverförmige Rinde, Cellulosepulver, mineralische Füllstoffe wie Perlit, synthetische Füllstoffe wie Nylonpulver, Rayonpulver, Diatomerde, Bentonit, Kaolin, Zeolithe, Talk, Lehm, Asche, Kohlenstaub, Magnesiumsilikate, Dünger oder Mischungen der Substanzen. Hochdisperse pyrogene Kieselsäure wie sie unter dem Handelsnamen Aerosil von Evonik Degussa vertrieben wird ist bevorzugt.

In einer bevorzugten Ausführungsform des erfindungsgemäßen Verfahrens erfolgt das in Kontakt bringen der Superabsorberteilchen mit dem Fluid enthaltend das nicht vernetzte Polymer in Gegenwart eines auf einem Polyzucker oder einer Silizium-Sauerstoff beinhaltenden Verbindung oder einer Mischung von mindestens zwei davon basierenden Effektstoffes. Dabei kann der Effektstoff im Fluid enthalten sein oder aber vor dem in Kontakt bringen der Superabsorberteilchen mit dem Fluid mit den Superabsorberteilchen vermischt werden. Es ist auch möglich, dass der Effektstoff in einem weiteren Fluid F' gelöst oder dispergiert wird und in Form dieser Lösung oder Dispersion zusammen mit dem Fluid mit den Superabsorberteilchen in Kontakt gebracht wird. Dabei umfasst das Fluid F' neben dem Effektstoff vorzugsweise eine Flüssigkeit, wobei als Flüssigkeit Wasser sowie organische Lösemittel wie etwa Methanol oder Ethanol, oder aber Mischungen aus mindestens zwei davon, besonders bevorzugt sind, wobei Wasser als Flüssigkeit besonders bevorzugt ist.

Als Polyzucker kommen erfindungsgemäß alle dem Fachmann geläufigen Stärken und deren Derivate sowie Cellulosen und deren Derivate sowie Cyclodextrine in Betracht, wobei als Cyclodextrine vorzugsweise α-Cyclodextrin, β-Cyclodextrin, γ-Cyclodextrin oder Mischungen aus diesen Cyclodextrinen eingesetzt werden.

Als Silizium-Sauerstoff beinhaltende Verbindungen sind Zeolithe bevorzugt. Als Zeolithe können alle dem Fachmann bekannten synthetischen oder natürlichen Zeolithe eingesetzt werden. Bevorzugte natürliche Zeolithe sind Zeolithe aus der Natrolith-Gruppe Harmoton-Gruppe, der Mordenit-Gruppe, der Chabasit-Gruppe, der Faujasit-Gruppe (Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabazit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmotom, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS.

Als Kationen enthalten die in dem erfindungsgemäßen Verfahren eingesetzten Zeolithe vorzugsweise Alkalimetall-Kationen wie Li⁺, Na⁺, K⁺, Rb⁺, Cs⁺ oder Fr⁺ und/oder Erdalkalimetall-Kationen wie Mg²⁺, Ca²⁺, Sr²⁺ oder Ba²⁺.

Als Zeolithe können Zeolithe des sogenannten "mittleren" Typs eingesetzt werden, bei denen das SiO₂/AlO₂-Verhältnis kleiner als 10 ist, besonders bevorzugt liegt das SiO₂/AlO₂-Verhältnis dieser Zeolithe in einem Bereich von 2 bis 10. Neben diesen "mittleren" Zeolithen können weiterhin Zeolithe des "hohen" Typs eingesetzt werden, zu denen beispielsweise die bekannten "Molekularsieb"-Zeolithe des Typs ZSM sowie beta-Zeolith gehören. Diese "hohen" Zeolithe sind vorzugsweise durch ein SiO₂/AlO₂-Verhältnis von mindestens 35, besonders bevorzugt von einem SiO₂/AlO₂-Verhältnis in einem Bereich von 200 bis 500 gekennzeichnet.

Vorzugsweise werden die Zeolithe als Partikel mit einer mittleren Partikelgröße in einem Bereich von 1 bis 500 µm, besonders bevorzugt in einem Bereich von 2 bis 200 µm und darüber hinaus bevorzugt in einem Bereich von 5 bis 100 µm eingesetzt.

Die Effektstoffe werden in den erfindungsgemäßen Verfahren vorzugsweise in einer Menge in einem Bereich von 0,1 bis 50 Gew.-%, besonders bevorzugt in einem Bereich von 1 bis 40 Gew.-% und darüber hinaus bevorzugt in einer Menge in einem Bereich von 5 bis 30 Gew.-%, jeweils bezogen auf das Gewicht der Superabsorberteilchen, eingesetzt.

Als keimhemmende Mittel sind grundsätzlich alle gegen grampositive Bakterien wirksamen Stoffe bevorzugt, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'- Methylen-bis(6-brom-4-chlorphenol), 3-Methyl-4-(1-methylethyl)phenol, 2-Benzyl-4-chlorphenol, 3-(4- Chlorphenoxy)-1,2-propandiol, 3-lod-2-propinylbutylcarbamat, Chlorhexidin, 3,4,4'-Trichlorcarbonilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen TM CAT, Cognis GmbH, Düsseldorf/Deutschland). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.
Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethem zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, alpha -Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, beta-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien reduzieren durch Beeinflussung der Aktivität der ekkrinen Schweißdrüsen die Schweißbildung, und wirken somit Achselnässe und Körpergeruch entgegen. Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium- Zirkonium-Trichlorohydrat, Aluminium-Zirkonium-Tetrachlorohydrat, Aluminium-Zirkonium-Pentachlorohydrat und deren Komplexverbindungen z. B. mit Aminosäuren wie Glycin.

Zum Mischen bzw. Besprühen sind alle Vorrichtungen geeignet, die eine homogene Verteilung des Fluids auf oder mit den Superabsorberteilchen erlauben. Beispiele sind Lödige Mischer (hergestellt durch die Firma *Gebrüder Lödige Maschinenbau GmbH*), Gerickc Multi-Flux Mischer (hergestellt durch die Firma *Gericke GmbH*), DRAIS-Mischer (hergestellt durch die Firma *DRAIS GmbH* Spezialmaschinenfabrik Mannheim), Hosokawa Mischer (*Hosokawa Mokron Co., Ltd*.), Ruberg Mischer (hergestellt durch die Firma *Gebr. Ruberg GmbH* & *CO.KG Nieheim*), Hüttlin Coater (hergestellt durch die Firma *BWI Hüttlin GmbH Steinen*), Fließbetttrockner oder Sprühgranulatoren von AMMAG (hergestellt durch die Firma *AMMAG Gunskirchen, Österreich*) oder Heinen (hergestellt durch die Firma *A. Heinen AG Anlagenbau Varel*), Patterson-Kelly-Mischer, NARA-Schaufelmischer, Schneckenmischer, Tellermischer, Wirbelschichttrockner, Schugi-Mischer oder PROCESSALL.

Für das in Kontakt bringen in einer Wirbelschicht können alle dem Fachmann bekannten und geeignet erscheinenden Wirbelschichtverfahren angewandt werden. Beispielsweise kann ein Wirbelschichtcoater eingesetzt werden.

Einen weiteren zur Lösung der eingangs beschriebenen Aufgaben liefert ein Verbund, beinhaltend die erfindungsgemäßen Superabsorber bzw. die durch das erfindungsgemäße Verfahren erhältlichen Superabsorber und ein Substrat. Es ist dabei bevorzugt, dass die erfindungsgemäßen Superabsorber und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich umfasst, welcher den erfindungsgemäßen Superabsorber in einer Menge im Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm³, vorzugsweise mindestens 0,1 cm³ und am meisten bevorzugt mindestens 0,5 cm³ aufweist.

In einer besonders bevorzugten Ausführungsform des erfindungsgemäßen Verbundes handelt es sich um einen flächenförmigen Verbund, wie er in der WO 02/056812 A1 als "absorbent material" beschrieben ist. Der Offenbarungsgehalt der WO 02/056812 A1, insbesondere hinsichtlich des genauen Aufbaus des Verbundes, des Flächengewichtes seiner Bestandteile sowie seiner Dicke wird hiermit als Referenz eingeführt und stellt einen Teil der Offenbarung der vorliegenden Erfindung dar.

Einen weiteren Beitrag zur Lösung mindestens einer der Eingangs genannten Aufgaben liefert ein Verfahren zur Herstellung eines Verbundes, wobei die erfindungsgemäßen wasserabsorbierende Polymer bzw. die durch das erfindungsgemäße Verfahren erhältlichen Superabsorber und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Einen Beitrag zur Lösung mindestens einer der Eingangs genannten Aufgaben liefert auch ein Verbund erhältlich nach dem vorstehend beschriebenen Verfahren, wobei dieser Verbund vorzugsweise die gleichen Eigenschaften aufweist wie der vorstehend beschriebene, erfindungsgemäße Verbund.

Einen weiteren Beitrag zur Lösung mindestens einer der Eingangs genannten Aufgaben liefern chemische Produkte beinhaltend die erfindungsgemäßen Superabsorber oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen Superabsorber oder des erfindungsgemäßen Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierende Mittel oder Pflanzenschutzwirkstoffe liefern einen Beitrag zur Lösung mindestens einer der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

### TESTMETHODEN

Sofern nicht nachfolgend anders angegeben, werden die hierin erfolgten Messungen nach ERT-Verfahren. "ERT" steht für *EDANA Recommended Test* und "EDANA" für *European Disposable and Nonwoven Association.*

### Zentrifugenretentionskapazität (CRC Centrifuge Retention Capacity)

Die Zentrifugenretentionskapazität wird nach der von der EDANA (European Disposables and Nonwovens Association) empfohlenen Testmethode Nr. 441.2-02 "Centrifuge retention capacity" bestimmt.

### Bestimmung der "Free Swell Rate" (FSR)

Die Bestimmung der Absorptionsgeschwindigkeit erfolgte über die Messung der sogenannten *"Free Swell Rate - FSR"* gemäß dem in der EP-A-0 443 627 auf der Seite 12 beschriebenen Testverfahren.

### Bestimmung der Permeabilität

Die Bestimmung der Permeabilität erfolgt durch die Messung der sogenannten "Saline Flow Conductivity-SFC" gemäß dem in der WO-A-95/26209 beschriebenen Testverfahren.

Die nachfolgenden Beispiele dienen der näheren Erläuterung der Erfindung ohne sie jedoch darauf zu beschränken.

### Beispiel 1

Eine Monomerlösung bestehend aus 2480 g Acrylsäure, 3124 g Wasser, 14.92 g Polyethylenglykol-300-diacrylat, 11.94 g Monoallylpolyethylenglykol-450-monoacrylsäure-ester wird durch Spülen mit Stickstoff vom gelösten Sauerstoff befreit. Die Acrylsäure ist zu 70 Mol-% mit Natronlauge neutralisiert (in diesem Beispiel mit 1927 g 50%ige NaOH). Die Stickstoffspülung erfolgt über einen Zeitraum von ca. 10 Minuten. Während der Inertgasspülung werden 2.33 g Natriumperoxodisulfat in 50 g Wasser zur Monomerlösung hinzugefügt. Kurz vor Transfer der Monomerlösung in den Polymerisationsreaktor erfolgt die Zugabe von 0.54 g 35%ige Wasserstoffperoxidlösung in 50 g Wasser zu der mit Inertgas gespülten Monomerlösung. Hieran schließt sich der Transfer der Monomerlösung in den Polymersationsreaktor an, der im Inertgasgegenstrom durchgeführt wird. Der Polymerisationsreaktor besteht aus einem zweiwelligen, gleichläufig drehenden, diskontinuierlich betriebenen Knetreaktor (LIST 10 Liter CKR Knetreaktor). Der Reaktor wird vor Zugabe der Monomerlösung über eine Mantel- und Wellenheizung auf ca. 85°C Heizmanteltemperatur gehalten. Die Wellen des Reaktors drehen mit 30 Umdrehungen pro Minute. Sofort nach Überführung der Monomerlösung in den Reaktor werden 0.5 Gew. %. Natriumcarbonat (hergestellt im Fließbett - Spray- Granulationsverfahren, Partikelgröße 400-600µm) zu der Monomerlösung gegeben und durch die drehenden Wellen im Reaktionsraum verteilt. Hierauf erfolgt die Zugabe von 0.233 g Ascorbinsäure in 50 g Wasser als Polymerisationsinitiator. Eine exotherme Polymerisationsreaktion findet statt. Die adiabatische Endtemperatur liegt im Bereich von 105 °C bis 110°C und die Verweilzeit des Reaktionsgemisches beträgt 10 Minuten. Ohne weitere Zerkleinerungsschritte wird das entstandene Hydrogel im Labor- Umlufttrockenschrank 120 Minuten bei 150 °C getrocknet. Das getrocknete Polymerisat wird in einer Schneidmühle (2mm) zerkleinert. Typ: Retsch-Mühle Durch Siebung wird eine Partikelgröße des zerkleinerten, getrockneten Superabsorbers von 150 µm bis 850 µm erhalten.

| Verfahren | CRC (g/g) | FSR (g/g/s) |
|---|---|---|
| Beispiel 1 | 30,8 | 0.35 |

### Oberflächen-Nachvernetzung

Eine anschließende Oberflächen-Nachvernetzung wird mit getrockneten, gemahlenen und abgesiebten Polymerpartikeln (= Precursormaterial) aus Beispiel 1 vollzogen. Hierzu wird die Oberflächen-Nachvernetzungslösung, bestehend aus Beispiel 1a) Ethylencarbonat/ Wasser (1 bzw. 3 Gew.% bezogen auf Superabsorbermasse) oder Beispiel 1b) Ethylencarbonat/Wasser/Aluminiumsulfat/Aluminiumlactat (1/3/0.3/0.4 Gew.% bezogen auf Superabsorbermasse) auf das Precursormaterial mittels einer Einwegspritze aufgesprüht und im Krupps-Mixer vermengt. Es erfolgt eine Trocknung/Oberflächennachvernetzung im Labortrockenschrank entweder bei 170°C/90 Minuten(i) oder 180°C/30 Minuten (ii). Folgende CRC- und FSR-Werte wurden gemessen:

| Verfahren | CRC (g/g) | FSR (g/g/s) |
|---|---|---|
| Beispiel 1a) -i | 27.5 | 0.36 |
| Beispiel 1.a) -ii | 28.5 | 0.31 |
| Beispiel 1.b) -i | 26.4 | 0.29 |
| Beispiel 1.b) -ii | 27.7 | 0.30 |

### Beispiel 2

Es wird eine Monomerlösung bestehend aus 2560 g Acrylsäure, die zu 70 Mol-% mit Natronlauge neutralisiert wurde (1989 g 50%ige NaOH), 3363 g Wasser, 9,21 g Polyethylenglykol-300-diacrylat, 13,31 g Monoallylpolyethylenglykol-450-monoacrylsäure-ester angesetzt. Hiervon werden für eine Polymerisation 2 kg abgenommen. Die abgenommenen 2 kg der Monomerlösung werden durch Spülen mit Stickstoff vom gelösten Sauerstoff befreit. Die Stickstoffspülung wird ca. 15 Minuten lang durchgeführt. Während der Inertgasspülung werden 0.60 g Natriumperoxodisulfat in 10 g Wasser zur Monomerlösung hinzugefügt. Kurz vor Transfer der Monomerlösung in den Polymerisationsreaktor erfolgt die Zugabe von 0.14 g 35%ige Wasserstoffperoxidlösung in 10 g Wasser zu der mit Inertgas gespülten Monomerlösung. Hieran schließt sich der Transfer der Monomerlösung in den Polymersationsreaktor im Inertgasgegenstrom an. Der Polymerisationsreaktor besteht aus einem zweiwelligen, gleichläufig drehenden, diskontinuierlich betriebenen Knetreaktor. (LIST 2.5 Liter CRP Knetreaktor) Der Reaktor wird vor Zugabe der Monomerlösung über einen Mantel auf ca. 75°C Heizmanteltemperatur erwärmt und die Temperatur gehalten. Die Wellen des Reaktors werden mit 60 Umdrehungen pro Minute gedreht. Sofort nach Überführung der Monomerlösung in den Reaktor werden 0.5 Gew. %. Natriumcarbonat (hergestellt im Fließbett - Spray- Granulationsverfahren, Partikelgröße 400-600µm) zu der Monomerlösung gegeben und durch die drehenden Wellen im Reaktionsraum verteilt. Hieran werden 0.03 g Ascorbinsäure in 2 g Wasser als Polymerisationsinitiator hinzugefügt. Die Soll-Temperatur des Thermostaten des Heizkreislaufes der Reaktormantelheizung wird direkt nach Start der Reaktion auf 100 °C eingestellt. Eine exotherme Polymerisationsreaktion findet statt. Die adiabatische Endtemperatur liegt im Bereich von 105 °C bis 110°C. Die Verweilzeit des Reaktionsgemisches beträgt 10 Minuten. Ohne weitere Zerkleinerungsschritte wird das entstandene Hydrogel im Labor-Umlufttrockenschrank 120 Minuten bei 150 °C getrocknet. Das getrocknete Polymerisat wird in einer Schneidmühle der Firma Retsch (2mm) zerkleinert. Durch Siebung wird eine Partikelgröße des zerkleinerten, getrockneten Superabsorbers von 150 µm bis 850 µm erhalten.

| Verfahren | CRC (g/g) | FSR (g/g/s) |
|---|---|---|
| Beispiel 2 | 32,4 | 0.34 |

### Oberflächen-Nachvernetzung:

Eine anschließende Oberflächen-Nachvernetzung wird mit getrockneten, gemahlenen und abgesiebten Polymerpartikeln (= Precursormaterial) aus Beispiel 2 vollzogen. Hierzu wird die Oberflächen-Nachvernetzungslösung, bestehend aus Beispiel 2a) Ethylencarbonat/Wasser/ Aluminiumsulfat/Aluminiumlactat (1/3/0.3/0.4 Gew. % bezogen auf Superabsorbermasse) auf das Precursormaterial mittels einer Einwegspritze aufgesprüht und im Krupps-Mixer vermengt. Anschließend erfolgt dann eine Trocknung/Oberflächennachvernetzung im Labortrockenschrank entweder bei 170°C/90 Minuten (i) oder 180°C/30 Minuten (ii).

**Folgende FSR-Werte wurden gemessen:**

| Verfahren | CRC (g/g) | FSR (g/g/s) |
|---|---|---|
| Beispiel 2a) -i | 29.1 | 0.35 |
| Beispiel 2a) -ii | 28.9 | 0.34 |

### Beispiel 3

Es wird eine Monomerlösung bestehend aus 2560 g Acrylsäure, die zu 70 Mol-% mit Natronlauge neutralisiert worden ist (1989 g 50%ige NaOH), mit 3363 g Wasser, 9,21 g Polyethylenglykol-300-diacrylat, 13,31 g Monoallylpolyethylenglykol-450-monoacrylsäure-ester angesetzt. Hiervon werden für eine Polymerisation 2 kg abgenommen. Die abgenommenen 2 kg der Monomerlösung werden durch Spülen mit Stickstoff vom gelösten Sauerstoff befreit. Die Stickstoffspülung erfolgt ca. 15 Minuten lang. Während der Inertgasspülung werden 0.60 g Natriumperoxodisulfat in 10 g Wasser zur Monomerlösung hinzugefügt. Kurz vor Transfer der Monomerlösung in den Polymerisationsreaktor erfolgt die Zugabe von 0.14 g 35%ige Wasserstoffperoxidlösung in 10 g Wasser zu der mit Inertgas gespülten Monomerlösung. Dann erfolgt der Transfer der Monomerlösung in den Polymersationsreaktor im Inertgasgegenstrom. Der Polymerisationsreaktor besteht aus einem zweiwelligen, gleichläufig drehenden, diskontinuierlich betriebenen Knetreaktor. (LIST 2.5 Liter CRP Knetreaktor. Der Reaktor wird vor Zugabe der Monomerlösung über einen Mantel auf ca. 75°C Heizmanteltemperatur gehalten. Die Wellen des Reaktors werden mit 60 Umdrehungen pro Minute gedreht. Sofort nach Überführung der Monomerlösung in den Reaktor wird 1.0 Gew. % Natriumcarbonat (hergestellt im Fließbett - Spray-Granulationsverfahren, Partikelgröße 400-600µm) zu der Monomerlösung gegeben und durch die drehenden Wellen im Reaktionsraum verteilt. Hiernach werden 0.03 g Ascorbinsäure in 2 g Wasser als Polymerisationsinitiator hinzugefügt. Die Soll-Temperatur des Thermostaten des Heizkreislaufes der Reaktormantelheizung wird direkt nach Start der Reaktion auf 100 °C gestellt. Eine exotherme Polymerisationsreaktion findet statt. Die adiabatische Endtemperatur liegt im Bereich von 105°C bis 110°C. Die Verweilzeit des Reaktionsgemisches beträgt 10 Minuten. Ohne weitere Zerkleinerungsschritte wird das entstandene Hydrogel im Labor-Umlufttrockenschrank 120 Minuten bei 150 °C getrocknet. Anschließend wird das getrocknete Polymerisat in einer Schneidmühle der Firma Retsch (2mm) zerkleinert. Durch Siebung wird eine Partikelgröße des zerkleinerten, getrockneten Superabsorbers von 150 µm bis 850 µm erhalten.

| Verfahren | CRC (g/g) | FSR (g/g/s) |
|---|---|---|
| Beispiel 3 | 33.1 | 0.38 |

### Oberflächen-Nachvernetzung:

Eine anschließende Oberflächen-Nachvernetzung wird mit getrockneten, gemahlenen und abgesiebten Polymerpartikeln (= Precursormaterial) aus Beispiel 3 vollzogen. Hierzu wird die Oberflächen-Nachvernetzungslösung, bestehend aus Beispiel 3a) Ethylencarbonat / Wasser / Aluminiumsulfat / Aluminiumlactat (1/3/0.3/0.4 Gew. % bezogen auf Superabsorbermasse) auf das Precursormaterial mittels einer Einwegspritze aufgesprüht und im Krupps-Mixer vermengt. Anschließend erfolgt eine Trocknung/Oberflächennachvernetzung im Labortrockenschrank entweder bei 170°C/90 Minuten (i) oder 180°C/30 Minuten (ii).

**Folgende CRC- und FSR-Werte wurden gemessen:**

| Verfahren | CRC (g/g) | FSR (g/g/s) |
|---|---|---|
| Beispiel 3a) -i | 30,7 | 0,36 |
| Beispiel 3a) -ii | 31,4 | 0,35 |

### Beispiel 4

Es wird eine Monomerlösung aus NaOH und Acrylsäure mit einem Neutralisationsgrad von 70 mol% sowie einer Acrylsäure WS von 32% hergestellt. In dieser Monomerlösung werden 0,2% (bezogen auf Acrylsäure) Vernetzer Polyethylenglykol-300-diacrylat sowie 0,4% (bezogen auf Acrylsäure) Monoallylpolyethylenglykol-450-monoacrylsäure-ester als Vernetzer gelöst.
Die Monomerlösung wird in einem 3L Becherglas für 30 Minuten mit Stickstoff durchspült, um den gelösten Sauerstoff zu entfernen. Bei einer Temperatur von 4 °C werden die 0,5% granulierte Soda (400-600µm) zugegeben und im Anschluss die Polymerisation durch die aufeinanderfolgende Zugabe von 0,3 g Natriumperoxidisulfat in 10 g dest. Wasser, 0,07 g 35 %ige Wasserstoffperoxidlösung in 10 g destilliertes Wasser und 0,015 g Ascorbinsäure in 2 g dest. Wasser gestartet. Nachdem die Endtemperatur (ca. 100 °C) erreicht ist, wird das Gel mit einem Fleischwolf zerkleinert und 2 h bei 150 °C in einem Labor-Umlufttrockenschrank getrocknet. Das getrocknete Produkt wird grob zerstoßen, gemahlen und auf eine Kornfraktion von 150-850µm abgesiebt.

| Verfahren | CRC (g/g) | FSR (g/g/s) |
|---|---|---|
| Beispiel 4 | 30,9 | 0,31 |

### Oberflächen-Nachvernetzung:

Eine anschließende Oberflächen-Nachvernetzung wird mit getrockneten, gemahlenen und abgesiebten Polymerpartikeln (= Precursormaterial) aus Beispiel 4 vollzogen. Hierzu wird die Oberflächen-Nachvernetzungslösung, bestehend aus Beispiel 4a) Ethylencarbonat / Wasser / Aluminiumsulfat / Aluminiumlactat (1/3/0.3/0.4 Gew. % bezogen auf Superabsorbermasse) auf das Precursormaterial mittels einer Einwegspritze aufgesprüht und im Krupps-Mixer vermengt. Anschließend erfolgt eine Trocknung/Oberflächennachvernetzung im Labortrockenschrank bei 170°C/90 Minuten (i) oder 180°C/30 Minuten (ii).

**Folgende CRC- und FSR-Werte wurden gemessen:**

| Verfahren | CRC (g/g) | FSR (g/g/s) |
|---|---|---|
| Beispiel 4a)- i | 26,9 | 0,26 |
| Beispiel 4a)-ii | 27,5 | 0,27 |

Trotz der Zugabe des granulierten Natriumcarbonats zeigte sich keine Verbesserung des FSR-Wertes bei Verwendung von eines Topfmischers.

### Beispiel 5

Die Ansatzmengen und die Versuchsdurchführung einschließlich der Nachvernetzung erfolgt gemäß dem Beispiel 2 und 2a). Der einzige Unterschied ist die Zugabe des Natriumcarbonats (0.5 Gew.%) direkt nach Zugabe der Ascorbinsäure erfolgt.

| Verfahren | CRC (g/g) | FSR (g/g/s) |
|---|---|---|
| Beispiel 5, Precursor | 35,2 | 0,38 |
| Beispiel 5 a) - i | 31,3 | 0,29 |
| Beispiel 5 a)- ii | 31,2 | 0,25 |

Erfindungsgemäß wirkt der Zugabezeitpunkt sich auf den resultierenden FSR-Wert aus und sollte bevorzugt nach H₂O₂-Zugabe und vor Ascorbinsäurezugabe erfolgen.

### Beispiel 6 Vergleichsbeispiel

Die Ansatzmengen und die Versuchsdurchführung einschließlich der Nachvernetzung erfolgt gemäß dem Beispiel 2 und Beispiel 2a). Der Versuch wurde ohne die Zugabe von Natriumcarbonat durchgeführt.

| Verfahren | CRC (g/g) | FSR (g/g/s) |
|---|---|---|
| Beispiel 6, Precursor | 31,6 | 0,33 |
| Beispiel 6a)-i | 28,4 | 0,23 |
| Beispiel 6 a)-ii | 28,6 | 0,20 |

Das Vergleichsbeispiel zeigt, dass der FSR-Wert ohne die Zugabe von Natriumcarbonat keine erfindungsgemäße Erhöhung nach dem Nachvernetzungsschritt aufweist.

## Patentansprüche

1. Ein Verfahren zur Herstellung einer wasserabsorbierenden Polymerzusammensetzung, umfassend die Verfahrenschritte
(i) Mischen von
(α1) 0,1 bis 99,999 Gew.-%, bevorzugt 20 bis 98,99 Gew.-% und besonders bevorzugt 30 bis 98,95 Gew.-% polymerisierbaren, ethylenisch ungesättigten, säuregruppenhaltigen Monomeren oder deren Salze oder polymerisierbaren, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
(α2) 0 bis 70 Gew.-%, bevorzugt 1 bis 60 Gew.-% und besonders bevorzugt 1 bis 40 Gew.-% polymerisierbaren, ethylenisch ungesättigten, mit (α1) copolymerisierbaren Monomeren,
(α3) 0,001 bis 10 Gew.-%, bevorzugt 0,01 bis 7 Gew.-% und besonders bevorzugt 0,05 bis 5 Gew.-% eines oder mehrerer Vernetzer,
(α4) 0 bis 30 Gew.-%, bevorzugt 1 bis 20 Gew.-% und besonders bevorzugt 5 bis 10 Gew.-% wasserlöslichen Polymeren, sowie
(α5) 0 bis 20 Gew.-%, bevorzugt 0,01 bis 7 Gew.-% und besonders bevorzugt 0,05 bis 5 Gew.-% eines oder mehrerer Hilfsstoffe, wobei die Summe der Gewichtsmengen (α1) bis (α5) 100 Gew.-% beträgt
(ii) radikalische Polymerisation unter Vernetzung, um ein wasserunlösliches, wässriges unbehandeltes Hydrogel-Polymer zu bilden,
(iii) Trocknen des Hydrogel-Polymers,
(iv) gegebenenfalls Mahlen und Absieben der wasserabsorbierenden Polymers,
(v) Oberflächennachvernetzung des gemahlenen Hydrogel-Polymers und
(vi) Trocknung und Konfektionierung des wasserabsorbierenden Polymers,
wobei
der wässrigen Monomerlösung, vor der Zugabe des Initiators und dem Start der radikalischen Polymerisation, Blähmittel mit einer Partikelgröße von 100 µm bis 900 µm zugesetzt werden,
**dadurch gekennzeichnet, dass** das Verfahren in den Schritten i) und ii) in einem Knetreaktor erfolgt und das Blähmittel aus einem Granulat aus Sodapartikeln besteht, wobei das Blähmittel aus Sodapartikeln besteht, die einen Schichtaufbau aufweisen, wobei es sich hierbei um ein Natriumcarbonat handelt, das eine partikulare Struktur aufweist, die aus mehreren Schichten Natriumcarbonat und Natriumsulfat und/oder einer Hochtemperaturphase aus einem Doppelsalz aus Na₄(SO₄)₁₊ₙ(CO₃)₁₋ₙ mit n von 0 bis 0,5 besteht; diese Carbonatverbindung hat eine Korngrößenverteilung im Bereich von 100 bis 900µm, die Herstellung erfolgt mittels "fluidized bed buildup granulation".

2. Verfahren nach Anspruch 1 **dadurch gekennzeichnet, dass** im Schritt iii) vor dem Trocknen des Hydrogel-Polymers gegebenenfalls auch eine Zerkleinerung erfolgt.

3. Verfahren nach einem der vorhergehenden Ansprüche **dadurch gekennzeichnet**, das die Verfahrensschritte i) und ii) im Knetreaktor diskontinuierlich oder kontinuierlich erfolgen.

4. Verfahren nach einem der vorhergehenden Ansprüche **dadurch gekennzeichnet**, das die Verfahrensschritte i) und ii) gemäß Anspruch 1 bevorzugt diskontinuierlich im Knetreaktor erfolgen.

5. Verfahren nach einem der vorhergehenden Ansprüche **dadurch gekennzeichnet**, das der Knetreaktor diskontinuierlich oder kontinuierlich mit einem mindestens einwelligem System betrieben wird und das granulierte Natriumcarbonat in der Monomerlösung und dem Verfahrensschritt ii) gleichmäßig eingearbeitet wird.

6. Verbund, beinhaltend ein wasserabsorbierendes Polymer hergestellt nach einem der vorhergehenden Ansprüche.

7. Verfahren zur Herstellung eines Verbundes, wobei ein wasserabsorbierendes Polymer nach einem der vorhergehenden Ansprüche und einem Hilfsmittel miteinander in Kontakt gebracht werden.

8. Verbund, erhältlich nach einem Verfahren gemäß Anspruch 7.

9. Schäume, Formkörper, Fasern, Folien, Filme, Kabel, Dichtungsmaterialien, flüssigkeitsaufnehmende Hygieneartikel, Träger für pflanzen- und pilzwachstumregulierende Mittel, Verpackungsmaterialien, Bodenzusätze oder Baustoffe, beinhalten das wasserabsorbierende Polymer nach einem der Ansprüche 1 bis 5 oder den Verbund nach Anspruch 6 oder 8.

10. Verwendung des wasserabsorbierenden Polymers hergestellt nach einem der Ansprüche 1 bis 5 oder eines ein wasserabsorbierendes Polymers nach einem der Ansprüche 6 oder 8 enthaltenen Verbunds in Schäumen, Formkörpern, Fasern, Folien, Filmen, Kabeln, Dichtungsmaterialien, flüssigkeitsaufnehmenden Hygieneartikeln, Trägern für pflanzen- und pilzwachstumregulierenden Mitteln, Verpackungsmaterialien, Bodenzusätzen, zur kontrollierten Freisetzung von Wirkstoffen oder in Baustoffen.

## Claims

1. Process for producing a water-absorbing polymer composition, comprising the process steps of
(i) mixing
(α1) 0.1 to 99.999% by weight, preferably 20 to 98.99% by weight and more preferably 30 to 98.95% by weight of polymerized, ethylenically unsaturated monomers containing acid groups or salts thereof or polymerized, ethylenically unsaturated monomers including a protonated or quaternized nitrogen, or mixtures thereof, particular preference being given to mixtures including at least ethylenically unsaturated monomers containing acid groups, preferably acrylic acid,
(α2) 0 to 70% by weight, preferably 1 to 60% by weight and more preferably 1 to 40% by weight of polymerized, ethylenically unsaturated monomers copolymerizable with (α1),
(α3) 0.001 to 10% by weight, preferably 0.01 to 7% by weight and more preferably 0.05 to 5% by weight of one or more crosslinkers,
(α4) 0 to 30% by weight, preferably 1 to 20% by weight and more preferably 5 to 10% by weight of water-soluble polymers, and
(α5) 0 to 20% by weight, preferably 0.01 to 7% by weight and more preferably 0.05 to 5% by weight of one or more assistants, where the sum of their weights (α1) to (α5) is 100% by weight,
(ii) free-radical polymerization with crosslinking to form a water-insoluble aqueous untreated hydrogel polymer,
(iii) drying the hydrogel polymer,
(iv) optionally grinding and sieving the water-absorbing polymer,
(v) surface postcrosslinking the ground hydrogel polymer and
(vi) drying and finishing the water-absorbing polymer,
wherein
blowing agents having a particle size of 100 µm to 900 µm are added to the aqueous monomer solution prior to the addition of the initiator and the start of the free-radical polymerization,
**characterized in that** the process in steps i) and ii) is effected in a kneading reactor and the blowing agent consists of granules of soda particles, wherein the blowing agent consists of soda particles having a layer structure,
where the soda here is a sodium carbonate having a particulate structure which consists of several layers of sodium carbonate and sodium sulphate and/or a high-temperature phase composed of a double salt of Na₄(SO₄)₁₊ₙ(CO₃)₁₋ₙ where n is from 0 to 0.5; this carbonate compound has a particle size distribution in the range from 100 to 900 µm; production is effected by means of fluidized bed buildup granulation.

2. Process according to Claim 1, **characterized in that**, in step iii), a comminution is optionally also effected prior to the drying of the hydrogel polymer.

3. Process according to either of the preceding claims, **characterized in that** process steps i) and ii) are effected batchwise or continuously in the kneading reactor.

4. Process according to any of the preceding claims, **characterized in that** process steps i) and ii) according to Claim 1 are preferably effected batchwise in the kneading reactor.

5. Process according to any of the preceding claims, **characterized in that** the kneading reactor is operated batchwise or continuously with an at least one-shaft system and the granulated sodium carbonate is incorporated homogeneously in the monomer solution and process step ii).

6. Composite including a water-absorbing polymer produced according to any of the preceding claims.

7. Process for producing a composite, wherein a water-absorbing polymer according to any of the preceding claims and an assistant are contacted with one another.

8. Composite obtainable by a process according to Claim 7.

9. Foams, mouldings, fibres, foils, films, cables, sealing materials, liquid-absorbing hygiene articles, carriers for plant growth and fungal growth regulators, packaging materials, soil additives or building materials, include the water-absorbing polymer according to any of Claims 1 to 5 or the composite according to Claim 6 or 8.

10. Use of the water-absorbing polymer produced according to any of Claims 1 to 5 or of a composite comprising a water-absorbing polymer according to either of Claims 6 and 8 in foams, mouldings, fibres, foils, films, cables, sealing materials, liquid-absorbing hygiene articles, carriers for plant growth and fungal growth regulators, packaging materials, soil additives, for controlled release of active ingredients or in building materials.

## Revendications

1. Procédé pour la préparation d'une composition de polymère absorbant l'eau, comprenant les étapes de procédé de
(i) mélange de
(α1) 0,1 à 99,999% en poids, de préférence 20 à 98,99% en poids et de manière particulièrement préférée 30 à 98,95% en poids de monomères polymérisables, éthyléniquement insaturés, contenant des groupes acides ou leurs sels ou de monomères polymérisables, éthyléniquement insaturés, contenant un azote protoné ou quaternisé ou leurs mélanges, Les mélanges contenant au moins des monomères éthyléniquement insaturés, contenant des groupes acides, de préférence l'acide acrylique étant particulièrement préférés,
(α2) 0 à 70% en poids, de préférence 1 à 60% en poids et de manière particulièrement préférée 1 à 40% en poids de monomères polymérisables, éthyléniquement insaturés, copolymérisables avec (α1),
(α3) 0,001 à 10% en poids, de préférence 0,01 à 7% en poids et de manière particulièrement préférée 0,05 à 5% en poids d'un ou de plusieurs réticulants,
(α4) 0 à 30% en poids, de préférence 1 à 20% en poids et de manière particulièrement préférée 5 à 10% en poids de polymères solubles dans l'eau, ainsi que
(α5) 0 à 20% en poids, de préférence 0,01 à 7% en poids et de manière particulièrement préférée 0,05 à 5% en poids d'un ou de plusieurs adjuvants, la somme des quantités pondérales (α1) à (α5) valant 100% en poids,
(ii) polymérisation par voie radicalaire avec réticulation, pour former un polymère sous forme d'un hydrogel aqueux, non traité, insoluble dans l'eau,
(iii) séchage du polymère sous forme d'hydrogel,
(iv) le cas échéant broyage et tamisage du polymère absorbant l'eau,
(v) postréticulation en surface du polymère sous forme d'hydrogel broyé et
(vi) séchage et confection du polymère absorbant l'eau, la solution aqueuse de monomères étant additionnée, avant l'addition de l'initiateur et le démarrage de la polymérisation par voie radicalaire, d'agent gonflant présentant une grosseur de particule de 100 µm à 900 µm, **caractérisé en ce que** le procédé dans les étapes i) et ii) a lieu dans un réacteur malaxé et l'agent gonflant est constitué par un granulat de particules de soude, l'agent gonflant étant constitué de particules de soude qui présentent une structure à couches, où il s'agit d'un carbonate de sodium qui présente une structure particulaire, qui est constituée par plusieurs couches de carbonate de sodium et de sulfate de sodium et/ou d'une phase haute température d'un double sel de Na₄(SO₄)₁₊ₙ(CO₃)₁₋ₙ, n valant 0 à 0,5 ; ce composé de carbonate présentant une distribution des grosseurs de particule dans la plage de 100 à 900 µm, la préparation ayant lieu au moyen d'une "fluidized bed buildup granulation" (granulation par accroissement en lit fluidisé).

2. Procédé selon la revendication 1, **caractérisé en ce qu'**un broyage a le cas échéant également lieu dans l'étape iii) avant le séchage du polymère sous forme d'hydrogel.

3. Procédé selon l'une quelconque des revendications précédentes, **caractérisé en ce que** les étapes de procédé i) et ii) ont lieu de manière discontinue ou continue dans le réacteur malaxé.

4. Procédé selon l'une quelconque des revendications précédentes, **caractérisé en ce que** les étapes de procédé i) et ii) selon la revendication 1 ont de préférence lieu de manière discontinue dans le réacteur malaxé.

5. Procédé selon l'une quelconque des revendications précédentes, **caractérisé en ce que** le réacteur malaxé est actionné de manière continue ou discontinue à l'aide d'un système présentant au moins un arbre et le carbonate de sodium granulé est incorporé de manière régulière dans la solution de monomères et dans l'étape de procédé ii).

6. Composite, contenant un polymère absorbant l'eau préparé selon l'une quelconque des revendications précédentes.

7. Procédé pour la préparation d'un composite, un polymère absorbant l'eau selon l'une quelconque des revendications précédentes et un adjuvant étant mis en contact l'un avec l'autre.

8. Composite pouvant être obtenu selon un procédé selon la revendication 7.

9. Mousses, corps façonnés, fibres, feuilles, films, câbles, matériaux d'étanchéité, articles hygiéniques absorbant les liquides, supports pour les agents régulant la croissance des plantes et des champignons, matériaux d'emballage, additifs pour sol ou matériaux de construction contenant le polymère absorbant l'eau selon l'une quelconque des revendications 1 à 5 ou le composite selon la revendication 6 ou 8.

10. Utilisation du polymère absorbant l'eau préparé selon l'une quelconque des revendications 1 à 5 ou d'un composite contenant un polymère absorbant l'eau selon l'une quelconque des revendications 6 ou 8 dans des mousses, des corps façonnés, des fibres, des feuilles, des films, des câbles, des matériaux d'étanchéité, des articles hygiéniques absorbant les liquides, des supports pour les agents régulant la croissance des plantes et des champignons, des matériaux d'emballage, des additifs pour sol, pour la libération contrôlée de substances actives ou dans des matériaux de construction.
